# EUROPEAN PATENT APPLICATION

(11) **EP 3 654 339 A1**
(43) Date of publication of application: **20.05.2020**
(21) Application number: 18206644.9
(22) Date of filing: 16.11.2018
(51) Int. Cl.: G16H 10/60, G16H 50/70

(54) **METHOD OF CLASSIFYING MEDICAL RECORDS**

(71) Applicant: Koninklijke Philips N.V., 5656 AG Eindhoven (NL)
(72) Inventor: LI, Zuofeng, 5656 AE Eindhoven (NL); WEN, Dong, 5656 AE Eindhoven (NL)
(74) Representative: de Haan, Poul Erik

(57) **Abstract**

A method for organizing medical record data based on classification of a set of medical records in accordance with an indexing intervention event identified for each record, associated with a medical intervention. The method is based on extracting for each of a plurality of medical records one or more candidate intervention events, and then mapping these to a dataset (or ontology) of standard intervention event names (indexing intervention events) in order to identify a closest matching indexing event for each extracted intervention event. The mapping is based on breaking down each extracted intervention event into a set of characterizing attributes of particular domains or types and then comparing these with corresponding attribute sets for each of the indexing events in the dataset. A closest match is found, and each medical record is classified according to the closest matching indexing event. Data is then aggregated based on the classifications, and also based on information about a user, e.g. a particular clinical area of expertise.

## Description

### FIELD OF THE INVENTION

The invention relates to a method for classifying medical records.

### BACKGROUND OF THE INVENTION

An increasing amount of data is now accrued in medical information systems. The systems are often poorly integrated, making review of patient information difficult and inefficient.

Typically patient data in for instance a hospital is primarily organized in accordance with the data source, such as a picture archive and communication system (PACS), a hospital information system (HIS), a radiology information system (RIS) and a laboratory information system (LIS). Compared to traditional paper-based medical records, information systems significantly improve organization and accessibility of data.

However, organization of information within the systems is often poorly structured, making it difficult for clinicians to find the information they need.

For example, physicians seeking to assess the current condition of a patient must access multiple different information system, and manually collate the data, which is inefficient. Furthermore, in the absence of context information, such as links to other of the patient's records, it is difficult for physicians to understand a patient's status in an intuitive way.

Furthermore, the increasing availability of very large volumes of patient data, leads to issues of information overload, where a clinician is unable to identify the specific information needed among the large quantity of available data. This can have potential negative consequences for patient outcome, such as errors or omissions, delays, and overall risks to patient safety.

Currently known patient information and display systems fail to meet the needs of clinicians as users. One example system which is used for instance is the Patient Holographic View. This is widely adopted and permits integration of data from various sources, and displays all information pertaining to a single patient in one page.

This addresses the issue of multiple entirely isolated sources of information, by connecting sources from different hospital information systems.

However, deficiencies still remain with such systems. In particular, because multiple information sources are linked, physicians are now presented with too much information to search and evaluate in an efficient matter. Hence the problem of information overload remains.

Furthermore, typically different physicians have different particular requirements in terms of the specific class of information they require. Also different kinds of information may be required in different circumstances.

For example, on first admittance of a patient for treatment, a physician may require examination and medication history information. Other information, such as demography information is not of use or relevance at this time.

An improved method of organizing medical record data is hence generally required.

### SUMMARY OF THE INVENTION

The invention is defined by the claims.

According to examples in accordance with an aspect of the invention, there is provided a method of classifying medical records, comprising:
obtaining a plurality of medical records;
processing the medical records in accordance with a data extraction model to extract from each record one or more intervention events, each representative of a medical intervention;
processing each of the derived intervention events in accordance with an algorithm to derive a representation of the event in terms of a set of characterizing attributes, the attributes comprising at least one attribute in each of a defined set of attribute domains;
accessing a dataset of indexing intervention events, each associated in the dataset with a corresponding representation in terms of a set of attributes, including at least one falling into each of said defined set of attribute domains, and based on comparison of the attributes of the extracted intervention events and the stored indexing intervention events, identifying a closest matching indexing event to each derived intervention event, and
classifying each of the medical records in accordance with the indexing event or events identified for that record;
selecting one of a plurality of indexing intervention events for use as a basis for aggregating the plurality of medical records, the selecting being based on information pertaining to a user; and
aggregating the obtained plurality of medical records on the basis of the selected indexing intervention event.

Embodiments of the invention are based on aggregating or organizing medical records based on different driving medical events (intervention events) to which the different records pertain.

An intervention event may refer for instance to a major medical intervention or treatment, and/or follow-up events subsequent to the intervention or treatment. In general, the intervention event may refer to a main medical event to which a number of records pertain. Various medical records may be associated with the same medical intervention event.

For example, these might include for instance the initial consultation in which a pathology is diagnosed and the referral for the particular curative intervention for curing the pathology. The curative intervention may be the intervention event in this case. Following this, follow-up consultations to monitor the condition may be classified in terms of a different intervention event, e.g. Outpatient Follow-Up. If there is recurrence of the pathology, records pertaining to this may be re-classified in terms of a different intervention event. Hence, the intervention event may be an event which characterizes an overall healthcare aim or purpose toward which records are directed or related.

By way of a specific example, a patient may be first diagnosed with liver cancer. Following this, he is referred for curative treatment in the form of a liver resection. The liver resection is the intervention event. Following referral, he is registered as an outpatient and the treatment is performed. All of these events may be classified in terms of the same intervention event (the liver resection)..Following this, there may be several follow-up outpatient consultations to monitor the patient condition. These may be classified differently, e.g. as Follow-Up Outpatient.

Embodiments of the invention are based on extracting from each medical record one or more candidate intervention events, for example based on a linguistic analysis technique, and then mapping this to one of a defined set of indexing events (indexing intervention events). This may be understood as mapping the extracted events to a defined intervention event ontology.

In order to perform the mapping, each extracted (derived) intervention event is first broken down or decomposed into a set of characterizing attributes, these falling into each of a defined set of attribute domains. The mapping is then based on comparison of the attributes of each extracted intervention event with attributes stored for the indexing intervention events, in order to find a closest matching indexing event for each extracted intervention event. This hence effectively maps each extracted event to one of the defined set of indexing events.

Each derived intervention event is then classified according to the identified closest matching indexing intervention event.

The classified records are then aggregated (e.g. sorted or organized) based on a selected one of the indexing intervention events. The selection of the indexing event on which basis to perform the aggregation is based on information pertaining to a user. This hence tailors the aggregation to the specific needs of a given user. For instance, the user information may be a clinical specialty or professional background of the user, which may indicate a particular one of the intervention events which is most relevant to his or her area of practice.

The data extraction model may in examples use language analysis techniques to extract the index events. The data extraction model may be trained in advance of the claimed method using a training procedure, the training procedure comprising selecting from each medical record a relevant subset of medical data, inputting the data to the model, and training the model in identifying a set of different index events from the data.

Conditional Random Field (CRF) or Convolution Neural Network (CNN) may be used for example to build the data extraction model.

The classifying may in examples comprise labeling the intervention event concerned.

Aggregating may mean grouping for example. For instance, all extracted intervention events which are classified with the selected indexing intervention event may be grouped together (aggregated), for viewing by a user in an organized fashion. Aggregating may hence mean organizing or sorting based on the classification.

Aggregation may further comprise filtering the extracted intervention events according to the selected indexing intervention event, i.e. filtering out from the extracted intervention events any events which have not been classified in accordance with the selected indexing intervention event.

The defined set of attribute domains may in certain examples include at least: an anatomical region to which the intervention event pertains, an intervention procedure to which the intervention event pertains, and a sub-type or category of said intervention procedure to which the intervention event pertains.

This choice of attribute domains has been found to be particularly efficient at organizing data in a powerful way.

The dataset of indexing intervention events may comprise an ontology of the indexing intervention events, the ontology defining links between each of the indexing intervention events and the associated sets of attributes. Ontology is a term of the art in the field of computer information technology. It encompasses for example a representation and formal naming of certain categories, properties, and relations between concepts that form part of a certain domain. For example, in the present case, the ontology may be for defining a set of standard intervention events (indexing intervention events) to which candidate events extracted from medical records may be mapped, based on attributes for the standard events stored in the ontology (as discussed above). The defined links may mean simply there being a respective set of attributes stored in the ontology dataset that is associated or linked with each the various indexing intervention event names in the dataset.

The aggregating of the medical records may comprise structuring the medical records into a hierarchical data structure, the hierarchical data structure comprising the obtained plurality of medical records grouped or sorted in accordance with the indexing event classification applied to each of the records.

According to one or more examples, the method may comprise a further step of determining for each indexing event classification of each medical record, a sub-classification, the sub-classification being based on a further attribute of the medical record concerned.

By way of example, in appropriate examples, the hierarchical data structure referred to above may have the obtained medical records further sorted, at a level subsidiary to that of the indexing event classification, according to a further attribute of the medical records. The subsidiary sorting level may be based on a sub-classification as determined in accordance with the above.

In certain examples for instance, the further attribute may comprise at least one of: a time-stamp of each medical record and a sub-category of the indexing event classification.

In this case, or according to any other example, the further attribute may be extracted from each medical record using a natural language processing tool.

The method may according to one or more examples, further comprise a training procedure for training the data extraction model, and the training procedure comprising selecting from the obtained plurality of medical records a subset of the medical records, inputting the selected subset of records to the model, and training the model for identifying a set of different index events from the data contained in said subset of records.

The training procedure may for instance be performed in advance of the step of processing the medical records.

According to certain examples, the training procedure may comprise use of a Conditional Random Field (CRF) or Convolutional Neural Network (CNN). Such tools may be used for example to build the data extraction model. Condition Random Fields and Convolution Neural Networks are well-known tools in the field of data processing, and the skilled reader will recognize the methods to which these terms refer.

The medical records may comprise text-based content linguistically representative of one or more intervention events, and wherein the data extraction model is configured to apply linguistic analysis methods for extracting the one or more intervention events.

The linguistic analysis technique may include a natural language processing technique.

The information pertaining to the user (referred to above) may in certain examples comprise identification information pertaining to the user, or information indicative of a clinical area of interest of the user.

Based on information indicative of a clinical area of interest, a most appropriate or relevant indexing intervention event may be selected as a basis for aggregating (i.e. grouping or sorting) the data. For instance, an indexing intervention event may be selected as one which is most clinically relevant to that clinical area of interest.

In the case that the information is identification information, here the identification information may be used to search or query a database which has stored certain preferred indexing intervention events for each user (linked to their respective identification information), or may simply have stored a clinical area of interest of each patient. This approach may be more efficient from the perspective of the user, since they need only input identification information and not a description of their clinical area of interest.

Hence, as noted, the selection of the indexing intervention event for performing the aggregation may in certain examples comprise querying a user database containing links between a plurality of users and a preferred indexing intervention event for each user.

According to one or more examples, the method may comprise selecting one of a plurality of stored data extraction models for performing the step of extracting the one or more intervention events, the data extraction model being selected based on information pertaining to a user.

The information pertaining to a user may for example be information indicative of a clinical area of interest and/or one or more preferred indexing intervention events. Based upon this, the method may select a data extraction model which is configured for extracting from the medical records (candidate) intervention events most relevant to that clinical area of the preferred indexing event. There may in certain examples be a data structure which stores for each available data extraction model a list of intervention events for which it is configured for extracting, and/or a list of indexing events to which it is configured for extracting.

Examples in accordance with a further aspect of the invention provide a computer program comprising code means for implementing the method according to any of the examples or embodiments outlined above, or described below, when said program is run on a computer.

Examples in accordance with a further aspect of the invention provide a processing unit, the processing unit configured to:
obtain a plurality of medical records;
process the medical records in accordance with a data extraction model to extract from each record one or more intervention events, each representative of a medical intervention;
process each of the extracted intervention events in accordance with an algorithm to derive a representation of the event in terms of a set of characterizing attributes, the attributes comprising at least one attribute in each of a defined set of attribute domains;
access a dataset of indexing intervention events, each associated in the dataset with a corresponding representation in terms of a set of attributes, including at least one falling into each of said defined set of attribute domains, and based on comparison of the attributes of the extracted intervention events and the stored indexing intervention events, identify a closest matching indexing event to each extracted intervention event; and
classify each of the medical records in accordance with the indexing intervention event or events identified for that record;
select one the indexing intervention events in the dataset for use as a basis for aggregating the plurality of medical records, the selecting being based on information pertaining to a user; and
aggregate the obtained plurality of medical records on the basis of the selected indexing intervention event.

Features of any of the examples, options or embodiments described above in relation to the method aspect of the invention may be applied with equal advantage to the above apparatus aspect of the invention.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which
Fig. 1 shows a block diagram of an example method according to one or more embodiments of the invention;
Fig. 2 schematically depicts an example workflow of one example method in accordance with one or more embodiments; and
Fig. 3 shows a block diagram of an example computer for use in implementing an example processing unit in accordance with one or more embodiments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for organizing medical record data based on classification of a set of medical records in accordance with an indexing intervention event identified for each record, associated with a medical intervention. The invention is based on extracting for each of a plurality of medical records one or more candidate intervention events, and then mapping these to a dataset (or ontology) of standard intervention event names (indexing intervention events) in order to identify a closest matching indexing event for each extracted intervention event. The mapping is based on breaking down each extracted intervention event into a set of characterizing attributes of particular domains or types and then comparing these with corresponding attribute sets for each of the indexing events in the dataset. A closest match is found, and each medical record is classified according to the closest matching indexing event. Data is then aggregated based on the classifications, and also based on information about a user, e.g. a particular clinical area of expertise.

Embodiments of the present invention are aimed at providing a more efficient means of aggregating and combining data from multiple different data sources, in a way that intelligently takes into account the requirements of different particular physicians.

In particular, embodiments of the invention may be understood as addressing at least two significant problems with current medical data systems.

First, it is highly inefficient for clinicians to seek specific clinical information, relevant to their practice, based on manually searching multiple disconnected medical records spread over multiple data source.

Medical records are typically scattered across different information systems. Despite recent improvements in the area of hospital information management data accessibility, records remain disjointed and poorly organized. It hence remains inconvenient and inefficient for physicians to identify relevant information, in particular due to poor links between associated records.

Furthermore, since different hospital information systems typically operate on different protocols, with different specific aims, direct communication or integration between systems is difficult. It requires inefficient manual intervention to group together records for a specific patient for instance.

Although a physician might for instance reduce these problems over time for a specific system through long-term usage and experience of the system (rendering data searching faster), when they come to review cases in other hospitals, it is necessary to learn how to use a new system.

A second main problem is that clinical staff with different roles or different clinical specialties may each have different specific data organization needs.

For example, physicians often have need to classify related clinical records for performing case review. In known Electric Medical Records (EMR) systems, clinical documents are typically sorted simply by chronology. Physicians must then use manual search and filter functions to acquire the records of selected patients, which is inefficient.

In different clinical scenarios, physicians may have particular information needs. A flexible classification for clinical documents would therefore be of value. Generally, physicians have need to compare and relate different records to analyze the status of patients.

To address the above problems, the present invention proposes a method of classifying and aggregating medical records (such as clinical documents) based on specific "driver events" to which each record can be associated. These driver events act as indexing events, since they are used to index or categorize different records for linking or aggregating.

The driver events, or indexing events, all relate to some clinical intervention or action, or occurrence. For this reason, they will be referred to as indexing intervention events.

The indexing intervention events, or driver events, in general represent some action or aim, or underlying 'driving' purpose behind each medical record. For instance, indexing intervention events may represent a major intervention (e.g. an operation), and records related to diagnosis, to hospital admittance, and to reports of the operation itself might be indexed to this intervention event. Following the operation, follow-up events, such as regular patient monitoring and clinician consultations may relate to a different indexing intervention event, since the driving aim is no longer the operation, but rather monitoring for stability and improvement.

By way of a specific example, a patient may first be diagnosed with liver cancer. In the case that they are fit for curative treatment (e.g. liver resection), such treatment would represent the indexing event for records leading up to the treatment. For instance, following diagnosis, the patient may be registered and admitted as an inpatient, and the treatment then performed. All of the activities leading up to the treatment and the treatment itself relate to the resection indexing intervention event.

After discharge, the relevant indexing (driver) event for subsequent medical records may change to Outpatient Follow-Up.

In the future, if the patient undergoes any reoccurrence of the pathology, the relevant indexing event may change to TACE (Transcatheter arterial chemoembolization) or another intervention.

All medical records relating to each of these different indexing events may be aggregated or clustered around the indexing events, as will be explained below.

It is noted that the specific general definition of what constitutes an indexing intervention event is not critical in a technical sense, since which events are classed as indexing intervention events may be inherently defined by the particular ontology, or indexing intervention event dataset, which is used (as will be explained below). The method according to embodiments involves matching or mapping all extracted candidate events to one of the indexing intervention events defined in this dataset or ontology, and hence this dataset effectively defines the set of indexing intervention events.

The advantage of classifying records based on these key intervention events is that clinicians from different disciplinary areas, and with different clinical interests, can easily sort or aggregate data according to particular kinds of intervention events which are relevant to them.

For example, in the case of a Multiple Disciplinary Team (MDT), experts from various departments may wish to see records from different perspectives for one patient. For example, for a liver cancer patient with hypertension, a cardiology expert may need to review records related to cardiovascular intervention events. The chronic disease history for the patient and abnormal vital signs may be significant factors for this user for instance.

However, a liver specialist may instead require information concerning the operation details of a liver resection procedure and for instance the progression of lab test results.

With the driver event based classification applied in embodiments of the present invention, each user is able to easily sort or aggregate records according to the particular intervention event classification which is relevant to them.

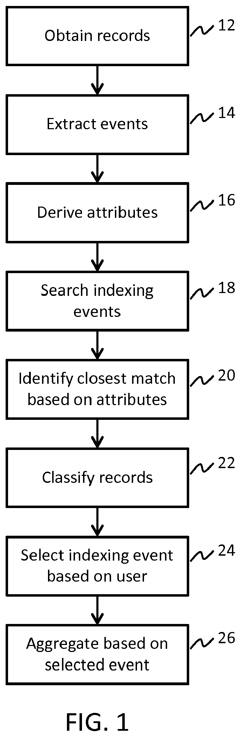

Fig. 1 illustrates an example method according to one or more embodiments of the present invention. The method will first be outlined in summary, to indicate the progression of steps, and then each specific step will be further explained and clarified in turn.

The example method comprises first obtaining 12 a plurality of medical records. The medical records may for instance be received as a data message from a remote computer, or the method may for example comprise actively accessing one or more data sources and retrieving or extracting the medical records. Other means of obtaining the records can also be used, as will be apparent to the skilled person.

The method further comprises processing 14 the medical records in accordance with a data extraction model to extract from each record one or more intervention events, each representative of a medical intervention. These intervention events may for instance be understood as candidate intervention events. The extraction may be based on natural language processing (NLP) techniques. For example, the medical records may each comprise text-based content (e.g. free text) linguistically representative of one or more intervention events, and wherein the data extraction model is configured to apply linguistic analysis methods for extracting the one or more intervention events.

The method further comprises processing 16 each of the extracted intervention events in accordance with an algorithm to derive a representation of the event in terms of a set of characterizing attributes, the attributes comprising at least one attribute in each of a defined set of attribute domains. The algorithm may be pre-determined and pre-stored, and configured for performing the extraction. This step involves breaking down or decomposing each extracted intervention event into a set of attributes falling into specific domains. By defining the required domains, this makes comparison of the event with events in the dataset of standard indexing events easier and more efficient, since it can be done based on their respective attributes in the common domains.

The method further comprises accessing a dataset of indexing intervention events, each associated in the dataset with a corresponding representation in terms of a set of attributes, including at least one falling into each of said defined set of attribute domains, and based on comparison 18 of the attributes of the extracted intervention events and the stored indexing intervention events, identifying 20 a closest matching indexing event to each extracted intervention event. This step hence represents a mapping of each extracted event to a standard set of indexing events in a dataset, the mapping being based on the attribute representations of the respective events. The dataset of indexing intervention events may represent an ontology of indexing intervention events.

Subsequent to identifying the closest matching indexing intervention event, the method comprises classifying 22 each of the medical records in accordance with the closest matching indexing intervention event or events identified for that record. Each record may be classified with more than one indexing intervention event, for instance if multiple intervention events are extracted for a given record, there may be a closest matching indexing event identified for each of these. Hence the record may be classified according to all of the closest matching indexing intervention events.

The method further comprises selecting 24 one of a plurality of indexing intervention events for use as a basis for aggregating the plurality of medical records, the selecting being based on information pertaining to a user. Here, the particular basis on which the medical records will be organized or grouped (i.e. aggregated) is selected. This is based on user-specific information, which may for instance relate to a clinical specialty of a clinician. In this way, data is organized or aggregated so that said records are grouped or sorted according to an indexing intervention event which is most relevant to the user concerned.

Accordingly, the method further comprises aggregating 26 the classified plurality of medical records on the basis of the selected indexing intervention event. The aggregating may for instance comprising grouping and/or sorting the records by the indexing intervention event selected. The aggregating may comprise filtering the records, to select only those records which are classified with the selected indexing intervention event.

These steps of the method will now be explained in greater detail below.

As discussed, embodiments of the invention are based on classifying medical records according a key driving event (indexing intervention event), to which each record pertains, where the indexing events on which the classifying is performed are defined in a standard stored dataset, or ontology.

As discussed, the indexing intervention events may be defined according to different underlying, or core, medical aims to which each record pertains. For instance, in the case of initial consultation at an outpatient stage, in some examples, the core (indexing) intervention event may be considered as diagnosis. In the case of a surgery in-patient event, the core intervention event may be considered to be the operation being performed.

For a different inpatient event, for instance an internal medicine inpatient event, the core intervention event may be considered to be the administered medication therapy.

Furthermore, since, in general, an overall intervention event can be related to multiple more specific treatment or diagnostic aims or events, according to one or more embodiments of the method, each indexing intervention event may be further divided into different event subtypes.

This allows for a further step in the method of determining for each indexing event classification applied to each medical record, a sub-classification, the sub-classification for example being based on a further attribute of the medical record concerned.

By way of example, the sub-classification may simply be based on a time stamp or tag of a particular record.

In further examples however, the sub-classification may relate to a more detailed or specific categorization of the intervention event concerned.

By way of a specific example, a lung resection intervention event may be subclassified as one of: complete resection, incomplete resection, uncertain resection and open and close operation. The sub-categorization may be performed based on semantic or linguistic analysis of the medical record concerned.

In the aggregation step, records may be further sorted, at a subsidiary level to that of the indexing intervention events, according to the designated sub-categorization.

In order to standardize the sub-categorizations, the dataset of indexing intervention events (otherwise known as an ontology of indexing intervention events) may comprise or encompass or define multiple sub-categorizations for some or all of the indexing intervention events included in the dataset.

As discussed, the invention is based on use of a dataset of indexing intervention events, wherein each extracted intervention event from each medical record is mapped or related to the indexing intervention events in the dataset based on comparison of a set of attributes of the events.

The dataset of indexing intervention events may represent or encompass or comprise an ontology of intervention events. This dataset or ontology effectively defines a set of standard intervention events (indexing intervention events) to which each intervention event extracted from each medical record may be mapped. This ensures that records can be sorted by a standard set of event names.

The method may in certain examples comprise a step of building a dataset of indexing intervention events. This dataset may constitute an indexing intervention event ontology. This may effectively be used as a seed library. An ontology is a well-known concept within the field of computer information science, and which in general represents a set of concepts which are organized in a tree structure.

The dataset or ontology of indexing intervention events may comprise for example a set of seed words, where these are pre-defined based on a clinical lexicon so as to be in accordance with the standard usage of clinical professional terms. These seed words may represent the names of each of the indexing intervention events.

For each indexing intervention event in the dataset, a set of characterizing attributes for the indexing intervention event is stored.

In one set of advantageous examples, this set of attributes comprises at least one attribute from each of a defined set of attribute domains.

Advantageously, the set of attributes may include one attribute in each of three specific attribute domains, these domains comprising: an anatomical region to which the intervention event pertains; an intervention procedure to which the intervention event pertains; and a sub-type or category of said intervention procedure to which the intervention event pertains. These three domains may be otherwise known as: the Feature domain, the Entity domain, and the Value domain. Entity refers to the anatomical region to which the intervention event pertains; Feature may refer to the key procedure such as a resection or other medical act or intervention; Value may refer to a detailed property or description of the event, i.e. a sub-category or type.

By way of a specific example, there exists a disease named Transcatheter arterial chemoembolization. It might be represented in terms of the above attribute domains as follows:
Entity domain: Arterial;
Feature domain: chemoembolization;
Value domain: operation.

The representation of each of the indexing intervention events in terms of such a set of attributes, for storing in the dataset or ontology, may be determined manually by a clinical expert for example. Alternatively, it may be determined automatically, for instance based on extraction of the key attributes from a textbook or other resource. This is optionally then subsequently reviewed by a clinical expert.

With the Entity-Feature-Value attribute breakdown of each indexing event in the ontology, one concept can be split into three parts, permitting the three attributes to be combined in different ways. The permits a broad range of categorizations of different intervention events in a very specific and flexible way. In this way, the expression of clinical concept knowledge can be expanded greatly to classify and sort unknown medical records through the combination of the three attribute domains as will be explained below.

The method according to embodiments involves a step of extracting from each medical record one or more intervention events. This is otherwise known as parsing the medical records. This is performed based on use of a data extraction model.

There may in certain examples be performed a process of building or training the data extraction models. This may either be done in advance of performing the method of the invention, or, in accordance with one or more embodiments of the invention, may be performed as an additional preliminary step in the method of the invention.

In either case, there may accordingly be performed a training procedure for training one or more data extraction models. This may be based for instance on selecting from the obtained plurality of medical records a subset of the medical records, inputting the selected subset of records to the model, and training the model for identifying a set of different intervention events from the data contained in said subset of records.

In accordance with one example, several data extraction models may be trained for extraction of candidate intervention events, i.e. to identify the name of an intervention event to which the record at least in part pertains. This may for instance include an operation name or therapy.

For each model which is built, first, a key sub-set of the plurality of medical records, or the data of the medical records, is selected. This may be based on selecting the key data which is most relevant to, or most indicative of, the particular intervention event(s) which the model concerned is to be configured for identifying and extracting.

The key data may for example comprise the data which represents the aim of each occurrence such as. the aim of a given visit to a consultant or clinician. The key data may be selected for instance from a full set of the medical records which were generated during a given visit to a clinician or hospital. By filtering down the medical records in this way, the training can be performed using only the most relevant data, which improves efficiency, and also the accuracy of the training.

For example, operation notes and pathology notes are important in the case of extracting or identifying a surgical intervention event. A progress note and a medical order may be important for detection of an inpatient treatment event. The selected subset of the data is then used for training the data extraction model to extract one or more intervention events.

In this training procedure, the input data is the selected medical records. The output is the intervention event name.

By way of example, a Conditional Random Field (CRF), or Convolutional Neural Network (CNN) may be used to build the data extraction model. Several intervention events may be extracted from a single medical record, or group of records. For example, for a group of records all relating to a particular visit to a clinician or medical center, multiple intervention events may be extracted from the records.

For example, a patient with coronary heart disease might be attending a hospital for a liver resection operation. Considering the pressure placed on the heart by this procedure, the doctor may administer coronary artery expansion therapy in advance of the main operation. Hence records will exist pertaining to the coronary artery expansion therapy, and for the main tumor resection therapy. For a physician whose clinical area of interest or specialty is the liver, the relevant intervention event is the liver tumor resection. However, for a physician whose clinical area of interest or expertise is cardiology, the most relevant intervention event is instead the coronary artery expansion.

Once one or more data extraction models have been built and/or trained (whether in advance of the method of the invention or as part of it), the model(s) can be applied to perform the step of extracting intervention events from the plurality of medical records.

As discussed, once one or more intervention event names (e.g. the operation name or the medication therapy name) has been extracted from the obtained plurality of medical records, it is necessary to map each of the extracted intervention events to a standard indexing intervention event listed in the common dataset or ontology.

This is based on transforming the operation name or medication therapy name into a representation in terms of a set of characterizing features, each belonging to one of a specific set of feature domains. The domains may be the Entity, Feature, Value domains discussed above. Hence in this case, each of the extracted intervention events is decomposed or broken down in into a corresponding 'Entity-Feature-Value' attribute pattern or representation. Thus for example, for each intervention event, a representation may be derived comprising a tuple or triple, consisting of the three attributes of the intervention event.

As noted, the Entity attribute refers for instance to the anatomic site to which the event pertains, the Feature attribute may correspond to the particular therapy or procedure type. The Value attribute may relate to different things, and corresponds in general to some more detailed property of the intervention event. For example, in some cases, it may refer to a particular material used.

For example, there exists an operation named percutaneous ethanol injection. Percutaneous indicates the anatomic site as the Entity attribute; injection indicates the procedure type as the Feature attribute; and ethanol indicates the therapy material as the Value attribute. Therefore, the intervention event can be mapped into a general pattern of three attributes.

It has already been discussed above that each indexing intervention event in the dataset or ontology is also stored with an associated representation in terms of characterizing attributes, for instance in terms of an Entity-Feature-Value pattern of attributes. This allows each extracted intervention event to be mapped to a closest matching standard indexing intervention event of the dataset based on comparison or mapping of the attribute set of the extracted event to attribute sets of the indexing events. This ensures that a common lexicon is used for referring to particular intervention event types, so that classification and aggregation of records is performed based on a common set of concepts.

For example, different names for the same anatomic site may by this process be merged.

According to certain examples, a sub-category of each intervention event may according to one or more examples be determined or extracted. This may for example be determined based on application of an NLP tool to each medical record. In this way, linguistic or sematic analysis is performed of the record and a sub-categorization determined based on this. By way of a specific example, in the case of for instance right lobe liver resection and bile duct resection, a specific indexing intervention event sub-classification of hepatobiliary resection operation may be derived.

For performing the comparison between the attribute set of the extracted intervention event and the attribute sets of the indexing intervention events stored in the dataset, in certain examples, a Levenshtein Distance algorithm may be used. This allows a similarity to be computed between any two sets of attributes, each pertaining to a common set of attribute domains for instance.

The Levenshtein distance is also known as the minimum edit distance. This in general permits measurement of the similarity between two strings. The distance corresponds to the number of deletions, insertions, or substitutions required to transform one string into another.

A closest matching indexing intervention event is determined for example as that whose associated attribute set exhibits the highest similarity level with the attribute set of the extracted intervention event. In the case of the Levenshtein distance algorithm, the highest similarity level corresponds to the shortest Levenshtein distance.

The medical record from which the relevant intervention event has been extracted may then be classified in accordance with the closest matching indexing intervention event(s).

As discussed, following this, classified medical records are aggregated based on the indexing event classifications. More particularly, the specific indexing intervention events by which the events are aggregated may be determined based on information pertaining to a user.

The indexing intervention event classifications thus provide a very efficient way of organizing a patient's medical history at a high level.

For example, the aggregating of the medical records may comprise structuring the medical records into a hierarchical data structure, the hierarchical data structure comprising the obtained plurality of medical records grouped or sorted in accordance with an indexing event classification applied to each of the records.

The hierarchical data structure may have the obtained medical records further sorted, at a level subsidiary to that of the indexing event classification, according to a further attribute of the medical records. For example, the medical records may be further sorted so as to follow the treatment timeline (i.e. chronology) of a patient.

The basis on which the records are aggregated or sorted may be selected in accordance with information pertaining to a user.

In some examples, the information pertaining to the user may comprise identification information pertaining to the user, or information indicative of a clinical area of interest of the user. It may be information pertaining to a clinical specialty of the user for example. It may be information pertaining to a professional (e.g. clinical) background of the user. In this way, the specific indexing intervention event upon which basis the records are sorted or aggregated may be selected based on context information about the user.

By way of example, the selection of the indexing intervention event for performing the aggregation may comprise querying a user database containing links between a plurality of users and a preferred indexing intervention event for each user.

In a given medical center for example, users (e.g. physicians) with different professional backgrounds and different clinical areas of interest may require aggregation and sorting of patient medical records in different ways.

For example, different clinicians may prefer data to be grouped or sorted or aggregated on the basis of different particular indexing intervention events, i.e. those events that are most relevant to their practice.

In some examples, a profile may be maintained for each of a number of users (e.g. clinicians), which indicates for instance the particular clinical area of interest or specialty of the user, and/or one or more specific indexing intervention events in which the user is most interested. Based on any of these factors, the method may select a particular indexing intervention event based upon which medical record aggregation should be performed.

In some examples, a profile may be maintained which takes into account a physician title, role, medical department, and/or details concerning the patient. The indexing intervention event upon which aggregation should be based may be selected based on this.

For example, for a physician from a cardiology department, practicing in a patient ward, an indexing intervention event relating to cardiovascular therapy may be selected.

The user profile may in any example be updated at certain intervals. This may be triggered for instance by interaction between the user and other applications being run on the given system.

As noted above, multiple data extraction models may be built in advance of running the method. In accordance with one or more embodiments, the method may comprise selecting one of a plurality of stored data extraction models for performing the step of extracting the one or more intervention events (from the medical records), the data extraction model being selected based on information pertaining to a user. The information pertaining to a user may for instance relate to a clinical area of interest of the user and/or one or more preferred indexing intervention events for aggregating data.

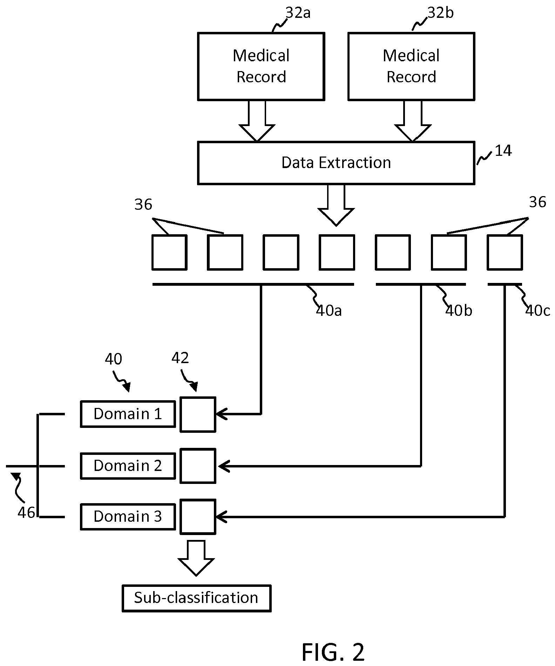

To illustrate the method further, Fig. 2 schematically depicts an example workflow of the method which will now be briefly outlined.

A plurality of medical records, originating from multiple data sources 32a, 32b are first obtained. These are then processed by a data extraction model in a data extraction step 14 in order to extract one or more intervention events to which each medical record pertains.

Following this, for each extracted intervention event, this is broken down into a representation in terms of a set of characterizing attributes 36, these attributes including at least one in each of a defined set of attribute domains 40a, 40b, 40c. In this case, there are three attribute domains. For example, these may correspond to the Entity-Feature-Value domains discussed above.

A single tuple 42, or set, of three attributes, one from each of the three domains is derived as a representation of each extracted intervention event. This is then mapped to a closest matching indexing intervention event stored in a dataset or ontology 48, based on comparison of the derived set 42 of attributes and sets of attributes stored in the dataset for different indexing intervention events.

Preferably, in addition to identifying a closest matching indexing intervention event, and classifying the extracted event based on this, also a sub-classification of the intervention event is also derived, this representing a more detailed or narrowed sub-category of the identified closest matching indexing intervention event.

Aggregation of the extracted intervention events (not shown) is then performed based on the applied categorizations and sub-categorizations.

Examples in accordance with a further aspect of the invention provide a processing unit, the processing unit configured to:
obtain a plurality of medical records;
process the medical records in accordance with a data extraction model to extract from each record one or more intervention events, each representative of a medical intervention;
process each of the extracted intervention events in accordance with an algorithm to derive a representation of the event in terms of a set of characterizing attributes, the attributes comprising at least one attribute in each of a defined set of attribute domains;
access a dataset of indexing intervention events, each associated in the dataset with a corresponding representation in terms of a set of attributes, including at least one falling into each of said defined set of attribute domains, and based on comparison of the attributes of the extracted intervention events and the stored indexing intervention events, identify a closest matching indexing event to each extracted intervention event; and
classify each of the medical records in accordance with the indexing intervention event or events identified for that record;
select one of the indexing intervention events in the dataset for use as a basis for aggregating the plurality of medical records, the selecting being based on information pertaining to a user; and
aggregate the classified plurality of medical records on the basis of the selected indexing intervention event.

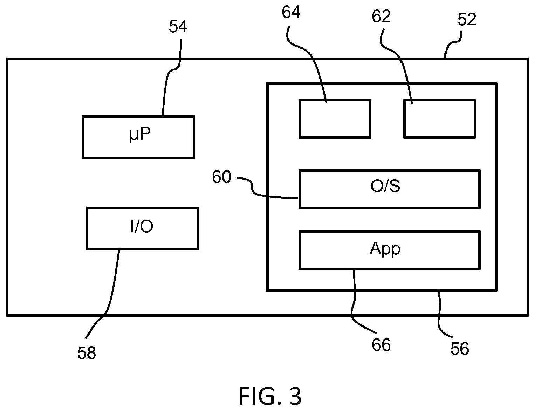

By way of example, Fig. 3 illustrates an example of a computer 52 for implementing the processing unit described above.

The computer 52 includes, but is not limited to, PCs, workstations, laptops, PDAs, palm devices, servers, storages, and the like. Generally, in terms of hardware architecture, the computer 52 may include one or more processors 54, memory 56, and one or more I/O devices 58 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 54 is a hardware device for executing software that can be stored in the memory 56. The processor 54 can be virtually any custom made or commercially available processor, a central processing unit (CPU), a digital signal processor (DSP), or an auxiliary processor among several processors associated with the computer 52, and the processor 54 may be a semiconductor based microprocessor (in the form of a microchip) or a microprocessor.

The memory 56 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 56 may incorporate electronic, magnetic, optical, and/or other types of storage media. Note that the memory 56 can have a distributed architecture, where various components are situated remote from one another, but can be accessed by the processor 54.

The software in the memory 56 may include one or more separate programs, each of which comprises an ordered listing of executable instructions for implementing logical functions. The software in the memory 56 includes a suitable operating system (O/S) 60, compiler 62, source code 64, and one or more applications 66 in accordance with exemplary embodiments.

The application 66 comprises numerous functional components such as computational units, logic, functional units, processes, operations, virtual entities, and/or modules.

The operating system 60 controls the execution of computer programs, and provides scheduling, input-output control, file and data management, memory management, and communication control and related services.

Application 66 may be a source program, executable program (object code), script, or any other entity comprising a set of instructions to be performed. When a source program, then the program is usually translated via a compiler (such as the compiler 62), assembler, interpreter, or the like, which may or may not be included within the memory 52, so as to operate properly in connection with the operating system 60. Furthermore, the application 66 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 58 may include input devices such as, for example but not limited to, a mouse, keyboard, scanner, microphone, camera, etc. Furthermore, the I/O devices 58 may also include output devices, for example but not limited to a printer, display, etc. Finally, the I/O devices 58 may further include devices that communicate both inputs and outputs, for instance but not limited to, a network interface controller (NIC) or modulator/demodulator (for accessing remote devices, other files, devices, systems, or a network), a radio frequency (RF) or other transceiver, a telephonic interface, a bridge, a router, etc. The I/O devices 58 also include components for communicating over various networks, such as the Internet or intranet.

When the computer 52 is in operation, the processor 54 is configured to execute software stored within the memory 56, to communicate data to and from the memory 56, and to generally control operations of the computer 52 pursuant to the software. The application 66 and the operating system 60 are read, in whole or in part, by the processor 54, perhaps buffered within the processor 54, and then executed.

When the application 66 is implemented in software it should be noted that the application 66 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method of classifying medical records, comprising:
obtaining (12) a plurality of medical records;
processing (14) the medical records in accordance with a data extraction model to extract from each record one or more intervention events, each representative of a medical intervention;
processing (16) each of the extracted intervention events in accordance with an algorithm to derive a representation of the event in terms of a set of characterizing attributes, the attributes comprising at least one attribute in each of a defined set of attribute domains;
accessing a dataset of indexing intervention events, each associated in the dataset with a corresponding representation in terms of a set of attributes, including at least one falling into each of said defined set of attribute domains, and based on comparison (18) of the attributes of the extracted intervention events and the stored indexing intervention events, identifying (20) a closest matching indexing intervention event to each extracted intervention event; and
classifying (22) each of the medical records in accordance with the indexing intervention event or events identified for that record;
selecting (24) one of a plurality of indexing intervention events for use as a basis for aggregating the plurality of medical records, the selecting being based on information pertaining to a user; and
aggregating (26) the classified plurality of medical records on the basis of the selected indexing intervention event.

2. A method as claimed in claim 1, wherein the defined set of attribute domains includes at least: an anatomical region to which the intervention event pertains, an intervention procedure to which the intervention event pertains, and a sub-type or category of said intervention procedure to which the intervention event pertains.

3. A method as claimed in claim 1, wherein the dataset of indexing intervention events comprises an ontology of the indexing intervention events, the ontology defining links between each of the indexing intervention events and the associated sets of attributes.

4. A method as claimed in claim 1, wherein the aggregating of the medical records comprises structuring the medical records into a hierarchical data structure, the hierarchical data structure comprising the obtained plurality of medical records grouped or sorted in accordance with the indexing event classification applied to each of the records.

5. A method as claimed in claim 4, wherein the hierarchical data structure has the obtained medical records further sorted, at a level subsidiary to that of the indexing event classification, according to a further attribute of the medical records.

6. A method as claimed in claim 5, wherein the further attribute comprises at least one of: a time-stamp of each medical record and a sub-category of the indexing event classification.

7. A method as claimed in claim 5, wherein the further attribute is extracted from each medical record using a natural language processing tool.

8. A method as claimed in claim 1, wherein the method further comprises a training procedure for training the data extraction model, and the training procedure comprising selecting from the obtained plurality of medical records a subset of the medical records, inputting the selected subset of records to the model, and training the model for identifying a set of different indexing intervention events from the data contained in said subset of records.

9. A method as claimed in claim 8, wherein the training procedure comprises use of a Conditional Random Field or Convolution Neural Network.

10. A method as claimed in claim 1, wherein the medical records comprise text-based content linguistically representative of one or more intervention events, and wherein the data extraction model is configured to apply linguistic analysis methods for extracting the one or more intervention events.

11. A method as claimed in claim 1, wherein the information pertaining to the user comprises either identification information pertaining to the user, or information indicative of a clinical area of interest of the user.

12. A method as claimed in claim 1 or 11, wherein the selection of the indexing intervention event for performing the aggregation comprises querying a user database containing links between a plurality of users and one or more preferred indexing intervention events for each user.

13. A method as claimed in claim 1, wherein the method comprises selecting one of a plurality of stored data extraction models for performing the step of extracting the one or more intervention events, the data extraction model being selected based on information pertaining to a user.

14. A computer program comprising code means for implementing the method of claim 1 when said program is run on a computer.

15. A processing unit, the processing unit configured to:
obtain a plurality of medical records;
process the medical records in accordance with a data extraction model to extract from each record one or more intervention events, each representative of a medical intervention;
process each of the extracted intervention events in accordance with an algorithm to derive a representation of the event in terms of a set of characterizing attributes, the attributes comprising at least one attribute in each of a defined set of attribute domains;
access a dataset of indexing intervention events, each associated in the dataset with a corresponding representation in terms of a set of attributes, including at least one falling into each of said defined set of attribute domains, and based on comparison of the attributes of the extracted intervention events and the stored indexing intervention events, identify a closest matching indexing event to each extracted intervention event; and
classify each of the medical records in accordance with the indexing intervention event or events identified for that record;
select one of the indexing intervention events in the dataset for use as a basis for aggregating the plurality of medical records, the selecting being based on information pertaining to a user; and
aggregate the classified plurality of medical records on the basis of the selected indexing intervention event.
